# EUROPEAN PATENT APPLICATION

(11) **EP 4 406 551 A1**
(43) Date of publication of application: **31.07.2024**
(21) Application number: 22872865.5
(22) Date of filing: 20.09.2022
(51) Int. Cl.: A61K 39/09, A61K 39/39, A61P 29/00, A61P 31/04, A61P 37/04, A61P 43/00, C07K 14/315, C07K 16/12

(54) **VACCINE INCLUDING NONENCAPSULATED STRAIN AS ANTIGEN**

(30) Priority: 21.09.2021 JP 2021152908
(71) Applicant: NATIONAL AGRICULTURE AND FOOD RESEARCH ORGANIZATION, Tsukuba-shi, Ibaraki 305-8517 (JP); School Corporation, Azabu Veterinary Medicine Educational Institution, Sagamihara-shi, Kanagawa 252-5201 (JP)
(72) Inventor: WATANABE, Atsushi, Sapporo-shi, Hokkaido 062-0045 (JP); HATA, Eiji, Tsukuba-shi, Ibaraki 305-0856 (JP); GOTO, Shinya, Sapporo-shi, Hokkaido 062-0045 (JP); KAWAI, Kazuhiro, Sagamihara-shi, Kanagawa 252-5201 (JP)
(74) Representative: Vossius & Partner Patentanwälte Rechtsanwälte mbB
(86) International application number: PCT/JP2022/034873
(87) International publication number: WO 2023/048111

(57) **Abstract**

It is an object of the present invention to provide a vaccine effective for protection against infection by Streptococcus species that causes mastitis in livestock and other animals. Specifically, the present invention provides a vaccine comprising a non-encapsulated strain of Streptococcus species as an antigen, and in particular, a vaccine for preventing mastitis developed by infection by Streptococcus uberis.

## Description

### Technical Field

The present invention relates to a vaccine, and in particular, to a vaccine for preventing infections caused by Streptococcus species.

### Background Art

Mastitis is a disease that frequently occurs in dairy farms, and is almost caused by bacterial infection in the udder. In particular, the incidence of mastitis caused by environmental streptococcus species is extremely high among all types of mastitis, and this is mastitis that occurs at the highest rate in Japan. Streptococcus uberis (hereinafter also referred to as "S. uberis"), Streptococcus dysgalactiae, etc. are known as environmental streptococcus species that cause mastitis. In particular, clinical mastitis caused by Streptococcus uberis is less responsive to antibiotic therapy, and for example, it may be repeatedly developed, resulting in refractory mastitis. Thus, Streptococcus uberis-derived mastitis has become one of the major dairy health problems, and thus, it has been desired to develop a novel method for controlling mastitis caused by environmental streptococcus species including Streptococcus uberis.

It would be considered that since the capsular antigenicity of Streptococcus uberis (hereinafter also referred to as "S. uberis") may differ among the strains, immunization with a certain type of S. uberis as a vaccine antigen is not effective against infection by other types of S. uberis. To date, various extracted antigen vaccines and component antigen vaccines have been proposed to protect against infection by S. uberis. For example, there is a report regarding an extracted antigen vaccine, in which a biofilm of S. uberis extracted by a heat treatment is used as an immunogen (Patent Literature 1). The vaccine using, as an immunogen, the biofilm of S. uberis extracted by a heat treatment, which is disclosed in Patent Literature 1, has a limited effect on a reduction in the number of somatic cells and the number of bacteria contained in milk, and this vaccine can suppress the progression to clinical mastitis, but it cannot eliminate intramammary infection of S. uberis. In addition, the component vaccines proposed so far also have limited efficacy.

As stated above, a vaccine for sufficiently protecting against infection by mastitis-causing Streptococcus species, in particular, with S. uberis, has not yet been reported.

### Citation List

### Patent Literature

Patent Literature 1: International Publication WO2017/140683

### Summary of Invention

### Technical Problem

In view of the above-described circumstances, it is an object of the present invention to provide a vaccine effective for protection against infection by Streptococcus species that cause mastitis in livestock and other animals.

### Solution to Problem

In order to achieve the aforementioned object, the present inventors have inoculated a dead bacterium of a S. uberis non-encapsulated strain used as an antigen (immunogen), together with an adjuvant, into cows, and as a result, the inventors have found that infection by not only the S. uberis non-encapsulated strain used as an antigen, but also infection by a S. uberis encapsulated strain that is different from the S. uberis non-encapsulated strain, was effectively protected.

That is to say, the present inventors have found that a vaccine containing a S. uberis non-encapsulated strain as an antigen protects against infection by non-encapsulated and capsulated S. uberis strains, without depending on capsular antigenicity, thereby completing the present invention.

Specifically, the present invention includes the following (1) to (11).
(1) A vaccine comprising a non-encapsulated strain of Streptococcus species as an antigen.
(2) The vaccine according to the above (1), which is characterized in that the antigen is a dead bacterium of the non-encapsulated strain or a portion thereof.
(3) The vaccine according to the above (1) or (2), which further comprises a pharmaceutically acceptable adjuvant.
(4) The vaccine according to any one of the above (1) to (3), which is for use in prevention of infection caused by Streptococcus species.
(5) The vaccine according to the above (4), wherein the infection is mastitis.
(6) The vaccine according to any one of the above (1) to (5), which is characterized in that the Streptococcus species is Streptococcus uberis (S. uberis).
(7) An antibody that binds to both of a non-encapsulated strain and an encapsulated strain of Streptococcus species.
(8) The antibody according to the above (7), which is characterized in that the antigen is a dead bacterium of the non-encapsulated strain of the Streptococcus species, or a portion thereof.
(9) The antibody according to the above (7) or (8), which is characterized in that the Streptococcus species is S. uberis.
(10) A method for preventing mastitis, comprising administering a pharmaceutically effective amount of a vaccine comprising a non-encapsulated strain of Streptococcus species as an antigen to a non-human mammal.
(11) The method according to the above (10), which is characterized in that the Streptococcus species is S. uberis.

It is to be noted that the preposition "to" sandwiched between numerical values is used in the present description to mean a numerical value range including the numerical values located left and right of the preposition.

### Advantageous Effects of Invention

The vaccine according to the present invention can effectively protect against infection by Streptococcus species, in particular, by S. uberis, without depending on the type of the bacterial strain.

### Brief Description of Drawings

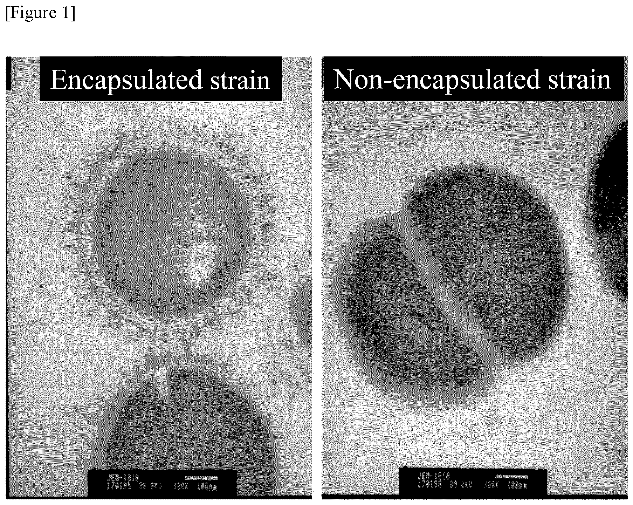

[Figure 1] Figure 1 shows electron microscopic images of a S. uberis encapsulated strain (left) and a S. uberis non-encapsulated strain (right).

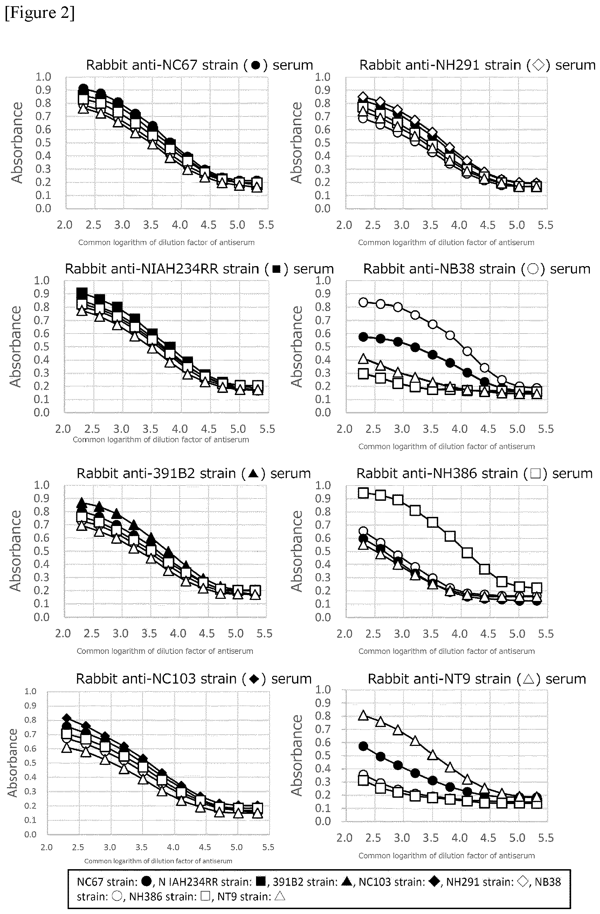

[Figure 2] Figure 2 shows the results obtained by examining the reactivity of a rabbit anti-S. uberis (non-encapsulated strains and encapsulated strains) antiserum against each strain according to an ELISA method. Dead bacteria of S. uberis non-encapsulated strains (NC67 ●, NIAH234RR ■, 391B2 ▲, NC103 ◆, and NH291 *) and encapsulated strains (NB38 o, NH386 □, and NT9 △) were immobilized in constant protein amounts on an ELISA plate, and the reactivity of an antiserum prepared against each strain was then examined.

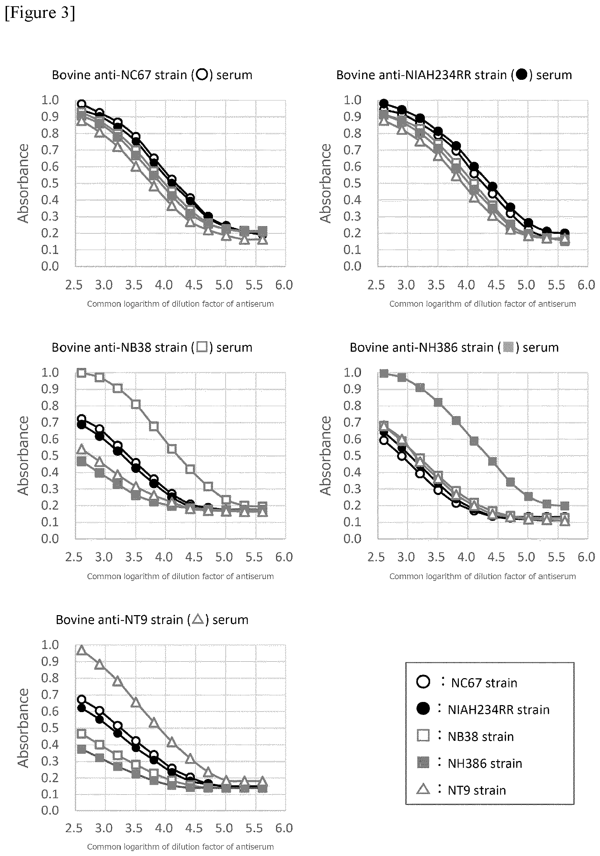

[Figure 3] Figure 3 shows the results obtained by examining the reactivity of a bovine anti-S. uberis (non-encapsulated strains and encapsulated strains) antiserum against each strain according to an ELISA method. Dead bacteria of S. uberis non-encapsulated strains (NC67 o and NIAH234RR ●) and encapsulated strains (NB38 □, NH386 ■, and NT9 △) were immobilized in constant protein amounts on an ELISA plate, and the reactivity of an antiserum prepared against each of the NC67 strain, the NIAH234RR strain, the NB38 strain, the NH386 strain, and the NT9 strain was then examined.

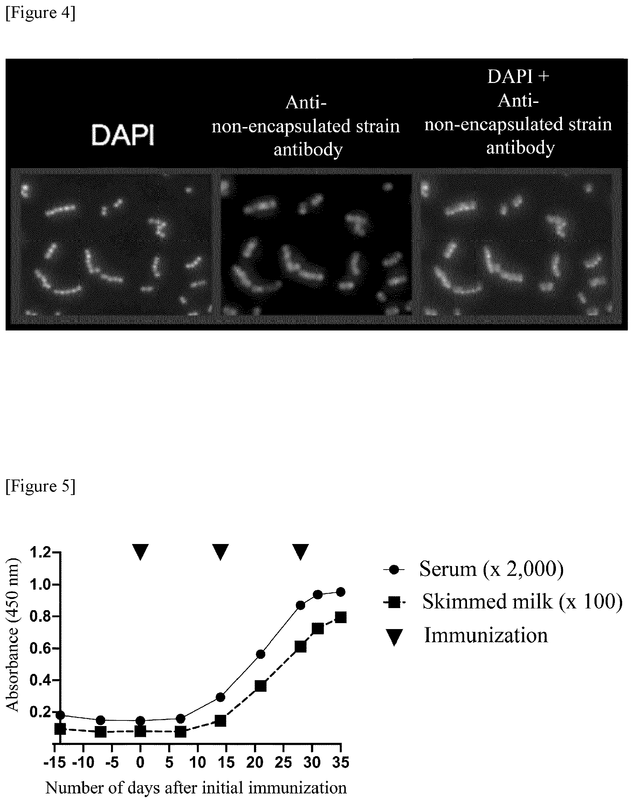

[Figure 4] Figure 4 shows the results of fluorescent immunostaining of the S. uberis encapsulated strain (NH386) with a bovine anti-S. uberis non-encapsulated strain antibody. Staining of nuclear DNA with DAPI (left), indirect fluorescent antibody staining with an anti-non-encapsulated strain antibody (antiserum) (center), and a merged image of the two types of staining (right) are shown.
[Figure 5] Figure 5 shows transitions in the anti-S. uberis IgG antibody titers in the serum (2,000-fold dilution) and skimmed milk (100-fold dilution) of milking cows immunized with a dead bacterium of the S. uberis non-encapsulated strain (NC67).

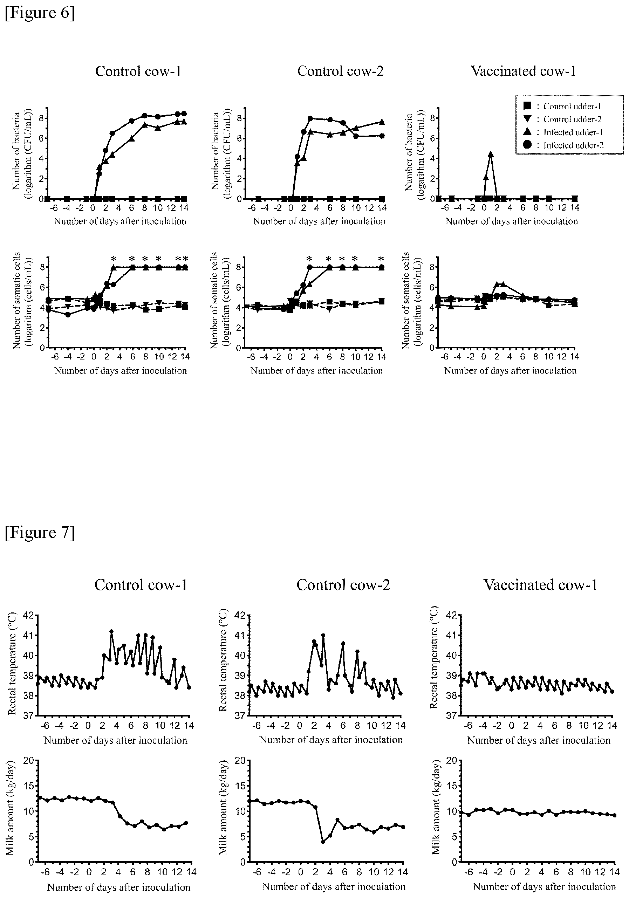

[Figure 6] Figure 6 shows studies (1) regarding the effects of vaccination with a S. uberis non-encapsulated strain as an immunogen against intramammary infection of S. uberis in lactating cows. Changes over time in the number of somatic cells and the number of bacteria in the milk of control cows and a vaccinated cow (a cow inoculated with a vaccine) are shown.
[Figure 7] Figure 7 shows studies (2) regarding the effects of vaccination with a S. uberis non-encapsulated strain as an immunogen against intramammary infection of S. uberis in lactating cows. Changes over time in the body temperature (rectal temperature) and milk amount of control cows and a vaccinated cow are shown.

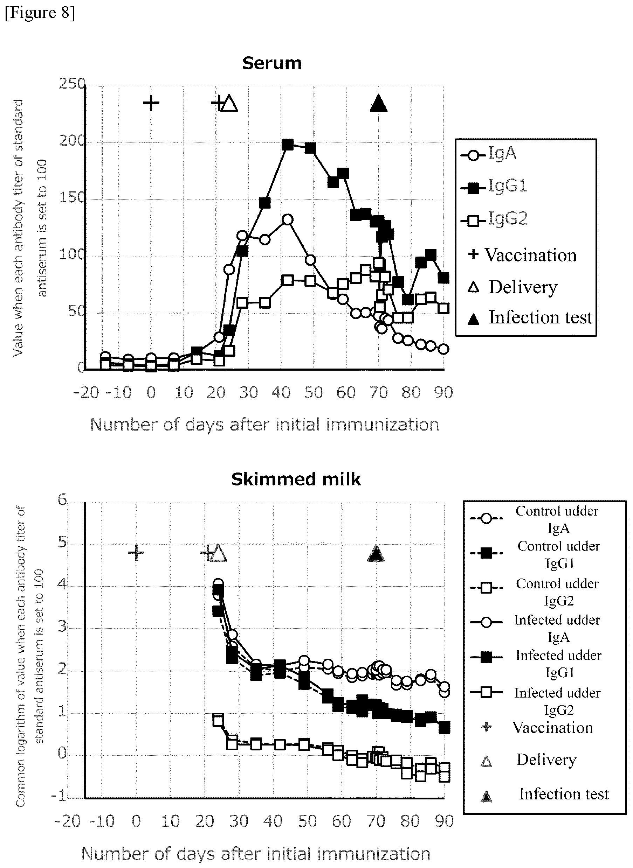

[Figure 8] Figure 8 shows studies (1) regarding the effects of vaccination against intramammary infection of S. uberis in lactating cows, in a case where a S. uberis non-encapsulated strain is inoculated as an immunogen into the lactating cows during the gestation period of the cows. Changes over time in the antibody titers of IgA, IgG1 and IgG2 in the serum (upper view) and skimmed milk (lower view) of the vaccinated cows are shown.

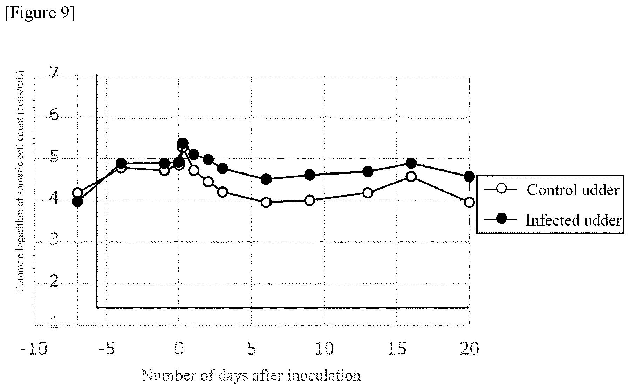

[Figure 9] Figure 9 shows studies (2) regarding the effects of vaccination against intramammary infection of S. uberis in lactating cows, in a case where a S. uberis non-encapsulated strain is inoculated as an immunogen into the lactating cows during the gestation period of the cows. Changes over time in the number of somatic cells in the milky juices from the infected udders and control udders of the vaccinated cows are shown.

### Description of Embodiments

Hereafter, the embodiments for carrying out the present invention will be described.

A first embodiment relates to a vaccine comprising a non-encapsulated strain of Streptococcus species as an antigen.

The Streptococcus species according to the present embodiment is not particularly limited, and it may be a species that infects into livestock or the like and develops mastitis therein. Examples of the Streptococcus species may include Streptococcus uberis and Streptococcus dysgalactiae, and in particular, Streptococcus uberis is desirable. In addition, the non-encapsulated strain (or non-encapsulated bacterium) is a bacterial strain that does not have a capsule and may be either a naturally occurring bacterial strain or an artificially produced bacterial strain.

Those skilled in the art can easily screen a naturally occurring Streptococcus species non-encapsulated strain from among Streptococcus species that have been separated and collected from the milk of livestock or the like with mastitis. Specifically, the bacteria of Streptococcus species are stained by a capsular staining method such as, for example, an India ink staining method, a hiss staining method and a method similar thereto (see, https://microbiologyinfo.com/capsule-staining-principle-reagents- procedure-and-result/ and https://www.austincc.edu/microbugz/capsule_stain.php, etc.), and the stained images are observed under an optical microscope, and then, a non-encapsulated strain of the Streptococcus species can be screened using the presence or absence of a capsule as an indicator. Finally, the presence or absence of a capsule is desirably confirmed by observation under an electron microscope.

Alternatively, such a non-encapsulated strains may also be screened by using the presence or absence of a gene involved in the formation of a capsule as an indicator. As such genes involved in the formation of a capsule, hasA (a gene encoding hyaluronan synthase) and hasC (a gene encoding UDP-glucose pyrophosphorylase ) are known (Accession Nos.: AJ242946 and AJ400707) (Ward et al., Infect. Immun. 69: 392-399 2001). If PCR is performed using genomic DNA extracted from a single colony of S. uberis as a template, and employing primers suitable for amplification of the above-described hasA or hasC, and if the amplified product is not obtained as a clear single band, the colony is highly likely to be a colony of a non-encapsulated strain (see, for example, Field et al., Infect. Immun. 71: 132-139 2003, etc.). Otherwise, the non-encapsulated strain may also be screened by confirming the presence or absence of a hasA or hasC gene according to Southern blotting or the like. Besides, when screening is carried out using, as an indicator, the presence or absence of a gene involved in capsule formation, it is desired to confirm the presence or absence of a capsule by electron microscopy, and to finally determine that it is a non-encapsulated strain.

The non-encapsulated strain of Streptococcus species can also be produced by deleting the function of a gene involved in the formation of a capsule in the encapsulated strain. In the case of S. uberis, for example, a non-encapsulated strain can be produced by, for example, introducing a mutation such as a deletion or insertion into hasA or hasC (see Ward et al., Infect. Immun. 69: 392-399 2001).

The vaccine according to the present embodiment comprises, as an antigen, an immunologically effective amount of a non-encapsulated strain of Streptococcus species (hereinafter, the "non-encapsulated strain of Streptococcus species" is also referred to as simply a "non-encapsulated bacterium" or "non-encapsulated strain" in the present description). The non-encapsulated strain comprised as an antigen in the vaccine of the present embodiment is an attenuated bacterium or dead bacterium that has lost the ability to infect biological tissues, or may be a constituent portion (e.g., a cell wall) of a dead bacterium of a non-encapsulated strain, or a constituent portion of a dead bacterium of a non-encapsulated strain, from which a portion possibly causing inconvenient inflammation that may result in side effects (e.g. DNA or lipoteichoic acid derived from the bacterium) has been eliminated. Herein, the term "immunologically effective amount" means the amount of a non-encapsulated strain of Streptococcus species that is required to induce an immune response sufficient to protect against infection by Streptococcus species in animals administered with the vaccine according to the present embodiment once or multiple times. Whether or not a sufficient immune response has been induced can be confirmed by using, as an indicator, the improvement of various signs evoked by mastitis in the vaccinated animals. An example of the signs evoked by mastitis may be, but is not particularly limited to, an increase in the number of bacteria or the number of somatic cells in the milk. Induction of a sufficient immune response can be confirmed, for example, based on whether or not the increased amount of bacteria or somatic cells is decrease to a desired amount. In the case of mastitis developed in livestock or the like by infection with Streptococcus species, the causative microorganisms enter the udder (for example, enter through the teat opening), then settle in the teat cistern, mammary gland cistern, milk ducts, mammary glands or the like in the udder, and then proliferate to form infection nests. Once such infected nests are formed, the causative microorganisms induce inflammation to the mammary gland tissues, and the milk contains neutrophils and somatic cells such as mammary gland epithelial cells shed by the inflammation, in addition to the causative microorganisms.

In addition, whether or not the amount of a non-encapsulated strain of Streptococcus species is an "immunologically effective amount" can also be evaluated by using, as an indicator, the degree of improvement of inflammation of mammary glands in vaccinated animals, the stability of the rectal temperature in the vaccinated animals, etc.

The vaccine according to the present embodiment may be administered to any mammals, preferably to cows, sheep, goats, camels, and the like. When the vaccine according to the present embodiment was administered to cows and the cows were then inoculated with an encapsulated strain of S. uberis, there was almost no increase in the number of bacteria and the number of somatic cells in the milk, compared with cows that were not inoculated with the vaccine. Also, the rectal temperature remained stable without fluctuation. Therefore, the vaccine according to the present embodiment exhibits effects in preventing the onset of infections caused by Streptococcus species, in particular, the onset of mastitis.

The vaccine according to the present embodiment may comprise a pharmaceutically acceptable adjuvant. The adjuvant used in the present embodiment is synonymous with the term "antigenic reinforcer" or "immunostimulant," etc., and the adjuvant of the present embodiment is used for the usual purposes of these agents in the present technical field. Examples of the adjuvant used in the embodiment of the present invention may include, but are not particularly limited to, a Freund's incomplete adjuvant, a Freund's complete adjuvant, aluminum salts (e.g., aluminum hydroxide, aluminum phosphate, aluminum sulfate, and aluminum chloride), oil in water emulsion (W/O type), water in oil (O/W type) emulsion, monophosphoryl lipid A (MPL), potassium alum, an adjuvant containing saponin, squalene, imidazoquinoline, muramyl dipeptide, trehalose dibehenate, dextran and the derivative thereof (such as DEAE dextran), biopolymers, chitosan, nonionic block copolymers, mineral oils, vegetable oils, and adjuvants containing a mixture of these substances. Preferred examples of the adjuvant of the present embodiment may include aluminum salts such as aluminum hydroxide and dextran derivatives such as DEAE dextran. The adjuvant of the present embodiment may be more preferably an adjuvant having immune enhancing effects that are equivalent to or greater than those of DEAE dextran. Moreover, the vaccine according to the present embodiment may also comprise a pharmacologically acceptable carrier. The pharmacologically acceptable carrier needs to be a compound that does not adversely affect the health of the animals to be vaccinated. Such a carrier is, for example, sterile water or a buffer.

The vaccine according to the present embodiment may comprise pharmaceutically acceptable known stabilizers, antiseptics, antioxidants, pH adjusters and preservatives. Examples of the stabilizer may include, but are not particularly limited to, gelatin, dextran, and sorbitol. Examples of the antiseptic may include thimerosal and β-propiolactone. The antioxidant may be, for example, α-tocopherol. Examples of the pH adjuster may include lactic acid, carbon dioxide, succinic acid, gluconic acid, citric acid, trisodium citrate, and potassium carbonate. Examples of the preservative may include paraoxybenzoic acid esters, phenol, and cresol.

The vaccine according to the present embodiment may comprise pharmaceutically acceptable additives, when it is formulated. The dosage form of the vaccine according to the present embodiment is not particularly limited, and it is, for example, a liquid formulation (e.g. nasal drops and an injection). When the vaccine according to the present embodiment is a liquid formulation, the active ingredient is mixed, as necessary, together with isotonic agents such as hydrochloric acid, sodium hydroxide, lactose, lactic acid, sodium, sodium chloride and glucose into distilled water for formulation, and thereafter, the obtained mixture is subjected to a sterilization treatment and is then filled into an ampule or the like. Otherwise, mannitol, dextrin, cyclodextrin, gelatin, etc. is further added to the mixture, and the thus obtained mixture is then subjected to vacuum-freeze drying, so as to make a ready-to-dissolve formulation upon use.

The vaccine according to the present embodiment may be administered by an intradermal, subcutaneous, intramuscular, intraperitoneal, intravenous, oral, transmucosal or intramammary administration route.

The dose of the vaccine according to the present embodiment can be determined, as appropriate, at the discretion of a physician or a veterinarian, taking into consideration the species of an animal, the age and body weight of a subject to be administered, etc., and the dose of the present vaccine is a pharmaceutically effective amount including an immunologically effective amount of antigen. For example, the vaccine according to the present embodiment may be administered at a single dose of vaccine antigen that is set at several µg to several tens of mg, once to several times a day, several times in total with intervals of 1 to several weeks, for example, 1 to 5 times, although the dose of the present vaccine is not particularly limited.

A second embodiment relates to a method for preventing mastitis, comprising administering a pharmaceutically effective amount (i.e. the amount of a vaccine exhibiting effects in protecting against infection of mastitis) of the vaccine according to the first embodiment (i.e. a vaccine containing, as an antigen, a non-encapsulated strain of Streptococcus species) to a mammal.

Herein, the term "prevention" is used to mean that the onset of mastitis is previously inhibited in a mammal that is likely to develop mastitis, and also mean a treatment for previously inhibiting the onset of mastitis. Furthermore, the treatment for inhibiting the recurrence of mastitis after the mastitis has been treated is also included in the "prevention."

The targets of prevention are not limited, as long as they are mammals, and examples thereof may include non-human mammals such as cows, sheep, and goats.

A third embodiment relates to an antibody that binds to both of a non-encapsulated strain and an encapsulated strain of the Streptococcus species.

The present inventors have found that an antibody produced using a non-encapsulated strain of Streptococcus species as an antigen binds to both of a non-encapsulated and an encapsulated strain. Accordingly, the antibody produced using a non-encapsulated strain of Streptococcus species (e.g. a dead bacterium of non-encapsulated strain) as an antigen can recognize not only the non-encapsulated strain, but also the encapsulated strain.

The antibody according to the present embodiment is not particularly limited in terms of the preparation method thereof and the structure thereof, and includes all "antibodies" that bind to desired antigens by desired properties, such as, for example, monoclonal antibodies, polyclonal antibodies, or nanoantibodies.

When the antibody according to the present embodiment is a polyclonal antibody, it can be prepared, for example, by inoculating a mixture of an antigen and an adjuvant into an animal to be immunized (although it is not limited, but it is, for example, a rabbit, a goat, sheep, a chicken, a guinea pig, a mouse, a rat or a pig, etc.). Usually, an antigen and/or an adjuvant are inoculated into the subcutis or abdominal cavity of an animal to be immunized multiple times. Examples of the adjuvant may include, but are not limited to, a complete Freund's adjuvant and monophosphoryl lipid A synthetic-trehalose dicolinomicolate (MPL-TMD). After immunization with the antigen, a desired antibody can be purified from the serum derived from the immunized animal according to a usual method (for example, a method using Protein A-retaining Sepharose, etc.).

On the other hand, when the antibody according to the present embodiment is a monoclonal antibody, it can be prepared, for example, as follows. Besides, the term "monoclonal" is used in the present description to suggest the properties of an antibody obtained from a substantially homogeneous population of antibodies (a population of antibodies, in which the amino acid sequences of the heavy and light chains constituting the antibodies are identical to one another), and thus, the term "monoclonal" does not mean that the antibody is produced by a specific method (e.g., a hybridoma method, etc.).

Examples of the method for producing a monoclonal antibody may include a hybridoma method (Kohler and Milstein, Nature 256: 495-497, 1975) and a recombination method (U.S. Patent No. 4,816,567).

The nanoantibody is a polypeptide consisting of the variable region of an antibody heavy chain (i.e. the variable domain of the heavy chain of a heavy chain antibody; VHH). In general, an antibody of a human or the like is composed of a heavy chain and a light chain. However, camelids such as llamas, alpacas and camels produce a single-chain antibody only consisting of a heavy chain (i.e., a heavy-chain antibody). Such a heavy-chain antibody can recognize a target antigen and can bind to the antigen, as in the case of an ordinary antibody consisting of a heavy chain and a light chain. The variable region of the heavy chain is a minimum unit having binding affinity for an antigen, and this variable domain fragment is referred to as a "nanoantibody." The nanoantibody has high heat resistance, digestion resistance and normal temperature stability, and thus, it is possible to more easily prepare a large amount of the nanoantibody according to a genetic engineering method.

The nanoantibody can be produced, for example, as follows. A camelid is immunized with an antigen, and then, the presence or absence of an antibody of interest is detected in the collected serum. Thereafter, cDNA is produced from RNA derived from the peripheral blood lymphocytes of the immunized animal in which a desired antibody titer is detected. A DNA fragment encoding VHH is amplified from the obtained cDNA, and is then inserted into a phagemid to prepare a VHH phagemid library. A desired nanoantibody can be produced from the prepared VHH phagemid library through several times of screening.

The antibody according to the present embodiment may be a genetically recombinant antibody. Examples of such a genetically recombinant antibody may include, but are not limited to, a human antibody, and a chimeric antibody with such a human antibody. The chimeric antibody means an antibody in which a variable region derived from an animal is ligated to a constant region derived from a different animal (for example, an antibody, in which a mouse-derived antibody variable region is ligated to a human-derived antibody constant region) and the like (e.g. Morrison et al., Proc. Natl. Acad. Sci. U.S.A. 81: 6851-6855 1984., etc.), and such a chimeric antibody can be easily constructed by a genetic recombination technique.

The humanized antibody is an antibody having a human-derived sequence in the framework region (FR) thereof and also having a complementarity determining region (CDR) consisting of a sequence derived from another animal species (e.g., a mouse, etc.). Using herein a mouse as an example of another animal species, the humanized antibody can be produced by first transplanting the CDRs of the variable region of an antibody derived from a mouse into a human antibody variable region, then reconstituting the heavy chain and light chain variable regions, and then linking these humanized reconstituted human antibody variable regions to human antibody constant regions. The methods for producing such a humanized antibody is well known in the present technical field (e.g. Queen et al., Proc. Natl. Acad. Sci. U.S.A. 86: 10029-10033, 1989, etc.).

The antigen-binding fragment of the antibody according to the present embodiment means a partial region of the antibody according to the present embodiment, which is an antibody fragment binding to an antigen. Examples of such an antigen-binding fragment may include Fab, Fab', F(ab')₂, Fv (a variable fragment of an antibody), a single-chain antibody (a heavy chain, a light chain, a heavy chain variable region, a light chain variable region, a nanoantibody, etc.), scFv (single chain Fv), a diabody (an scFv dimer), dsFv (disulfide-stabilized Fv), and a peptide comprising the CDR of the antibody according to the present embodiment in at least a portion thereof.

Fab refers to an antibody fragment having antigen-binding activity obtained by digesting an antibody molecule with the protease papain, wherein about an N-terminal half of the heavy chain binds to the entire light chain via a disulfide bond. Fab can be produced by digesting an antibody molecule with papain to obtain a fragment thereof. Fab can also be produced by, for example, constituting a suitable expression vector into which DNA encoding the Fab has been inserted, then introducing the vector into suitable host cells (e.g. mammalian cells such as CHO cells; yeast cells; insect cells; etc.), and then allowing the Fab to express in the cells.

F(ab')₂ refers to an antibody fragment having antigen-binding activity obtained by digesting an antibody molecule with the protease pepsin, which is slightly greater than Fab that binds to another Fab via a disulfide bond at hinge region. F(ab')₂ can be obtained by digesting an antibody molecule with the pepsin, or it can also be produced by binding Fab to another Fab via a thioether bond or a disulfide bond. Alternatively, F(ab')₂ can also be produced according to a genetic engineering method, as with Fab.

Fab' refers to an antibody fragment having antigen-binding activity obtained by cleaving the disulfide bond at hinge region of the above-described F(ab')₂. Fab' can also be produced according to a genetic engineering method, as in the case of Fab and the like.

scFv refers to an antibody fragment having antigen-binding activity that is a VH-linker-VL or VL-linker-VH polypeptide, wherein a single heavy chain variable region (VH) is ligated to a single light chain variable region (VL) using a suitable peptide linker. Such scFv can be produced by obtaining cDNAs encoding the heavy chain variable region and light chain variable region of an antibody and then treating them according to a genetic engineering method.

Diabody refers to an antibody fragment having divalent antigen-binding activity, in which scFv is dimerized. The divalent antigen-binding activity may be either two identical antigen-binding activities or two different antigen-binding activities. The diabody can be produced by obtaining cDNAs encoding the heavy chain variable region and light chain variable region of an antibody, then constructing cDNA encoding scFV, in which the heavy chain variable region is ligated to the light chain variable region using a peptide linker, and then treating the cDNA according to a genetic engineering method.

dsFv refers to a polypeptide, in which one amino acid residue each in the heavy chain variable region and the light chain variable region is replaced with a cysteine residue, and the polypeptides bind to each other a disulfide bond between the cysteine residues. The amino acid residue to be substituted with the cysteine residue can be selected based on prediction of the three-dimensional structure of antibodies. Such dsFv can be produced by obtaining cDNAs encoding the heavy chain variable region and light chain variable region of the antibody and then constructing DNA encoding the dsFv according to a genetic engineering method.

A peptide comprising CDR is constituted such that it comprises at least one region of CDRs (CDR1-3) of the heavy chain or the light chain. A peptide, which comprises multiple CDR regions, is able to bind to another peptide directly or via a suitable peptide linker. In the case of such a peptide comprising CDR, DNA encoding the CDR of the heavy chain or light chain of the antibody is constructed, and it is then inserted into an expression vector. The type of the vector is not particularly limited, and it may be selected, as appropriate, depending on the type of a host cells into which the vector is to be introduced, and the like. The vector is introduced into host cells suitable for the expression of an antibody (e.g. mammalian cells such as CHO cells, yeast cells, insect cells, etc.), so that the peptide can be produced. Alternatively, such a peptide comprising CDR can also be produced by chemical synthesis methods such as an Fmoc method (fluorenylmethyloxycarbonyl method) and a tBoc method (t-butyloxycarbonyl method).

A human antibody (complete human antibody) generally has the same structure as the antibody of a human, in terms of the structures of a hypervariable region as an antigen-binding site in the V region), other parts in the V region, and a constant region. The human antibody can be easily produced by those skilled in the art using known techniques. The human antibody can be obtained, for example, by a method using a human antibody-producing mouse having a human chromosome fragment containing the H- and L-chain genes of a human antibody (for example, Tomizuka et al., Proc. Natl. Acad. Sci. U.S.A. 97: 722-727, 2000, etc.) or a method of obtaining a human antibody derived from a phage display selected from a human antibody library (for example, Siriwardena et al., Opthalmology, 109: 427-431, 2002, etc.).

The antigen-binding fragment of the antibody according to the present embodiment can be used to construct a multispecific antibody. Multispecificity means that an antibody has binding specificity to two or more antigens, and such multi specificity may be, for example, the form of a protein comprising a monoclonal antibody or an antigen-binding fragment, having binding specificity to two or more antigens. This multi specificity can be achieved by those skilled in the art using known techniques. Several methods have been developed as methods for constituting multi specificity, and such methods can be classified into: techniques of constructing asymmetric IgG, in which protein engineering operations are performed on two different types of antibody heavy chain molecules to allow the molecules to form a heterodimer; techniques of linking antigen-binding fragments obtained from an antibody to each other or linking the antigen-binding fragment to another antibody molecule; and other techniques. As an example of a specific construction method, for example, the following publication can be referred to: Kontermann and Brinkmann, Drug Discov. Today, 20: 838-847, 2015.

When the present description is translated into English and the English description includes singular terms with the articles "a," "an," and "the," these terms include not only single items but also multiple items, unless otherwise clearly specified from the context that it is not the case.

Hereinafter, the present invention will be further described in the following examples. However, these examples are only illustrative examples of the embodiments of the present invention, and thus, are not intended to limit the scope of the present invention.

### Examples

### 1. Screening and identification of S. uberis non-encapsulated strain

First, various S. uberis strains were separated and collected from the milk of clinical type and latent mastitis developed in dairy farms in various parts of Japan. Specifically, the bacteria contained in mastitis milk (i.e. milk derived from inflammatory udders) were cultured on a sheep blood agar medium, and were then screened by using the shapes and properties of colonies formed on the medium as indicators. Thereafter, the bacteria were further screening by PCR using 16S rDNA as a target (Hassan et al., J. Clin. Microbiol. 39: 1618-1621, 2001). The 16S rDNA sequence of the bacterial strain obtained by the screening was amplified by PCR (Kuhnert et al., Int. J. Syst. Bacteriol. 46: 1174-1176, 1996), the nucleotide sequence of the amplified product was then determined. The obtained nucleotide sequence was compared with the nucleotide sequence of S. uberis HN1 16S rDNA (DDBJ/EMBL/GenBank accession number AB023576), and based on the homology, the nucleotide sequence was identified to be a S. uberis strain (Tomita et al., Appl. Environm. Microbiol. 74: 114- Microbiol. 74: 114- 124, 2008).

### 1-1. Screening of S. uberis non-encapsulated strain by PCR

First, the presence or absence of the hasA gene, which is required for capsule formation of S. uberis, was examined by a PCR method, and finally, the presence or absence of a capsule was confirmed by electron microscopy. The details are specifically described below.

The collected S. uberis was cultured on a sheep blood agar medium overnight at 37°C. Colonies of growing bacteria were picked up and were then subjected to shaking culture (200 rpm, 37°C, and 16 hours) in a Todd-Hewitt broth medium (e.g. Oxoid Ltd.). The culture was centrifuged (6,000 x g, 5 min), the supernatant was then removed, and the bacteria were then collected as precipitates. Using a commercially available DNA extraction kit (e.g., ISOPLANT II: NIPPON GENE CO., LTD., Toyama), DNA was purified from the collected bacteria. Using the purified bacterial DNA as a template, the primer hasA-for (5'-GAAAGGTCTGATGCTGATG-3': SEQ ID NO: 1) and the primer hasA-rev (5'-TCATCCCCTATGCTTACAG-3': SEQ ID NO: 2) were used to perform PCR on the capsular synthetic gene hasA as an amplification target (Field et al., Infect. Immun. 71: 132-139, 2003). The PCR reaction solution was subjected to agarose gel electrophoresis and was stained with a DNA staining reagent (ethidium bromide, etc.) to confirm the presence or absence of a band as an amplified product. Herein, the PCR amplified products were classified into (1) those with a clear single band, (2) those with no band at all, and (3) those with no clear single band (unclear band). The presence or absence of a capsule was confirmed by electron microscopic observation for the bacterial strains of the above (2) and (3), and as a result, no capsules were observed for all of the bacterial strains. As a comparative control, electron microscopic observation was also performed on strains for which a clear single band was obtained by PCR amplification and capsules could be easily observed by a capsular staining method (e.g., an India ink method) at the optical microscopic level.

### 1-2. Confirmation by electron microscopy

The electron microscopic observation was performed by the following procedures.

A S. uberis strain was cultured on a sheep blood agar medium at 37°C for 24 hours for proliferation, and bacterial colonies were obtained. The obtained bacterial colonies were scraped with an inoculation loop, were then subjected to centrifugal washing with PBS, and were then suspended in a fixative solution (a phosphate buffer containing 2.5% glutaraldehyde and 2% paraformaldehyde). The bacterial suspension was centrifuged, and the bacteria were collected as pellets. Subsequently, PBS containing 1.5% (w/v) low-melting-point agarose that was kept warm at 37°C was added to the recovered bacterial pellets, and the obtained mixture was gently mixed, so that the bacteria were embedded. Thereafter, the embedded bacteria were transferred to 4°C. The solidified agarose was cut into 1 to 2 mm² pieces, and the agarose pieces were then immersed in a fixative solution (a phosphate buffer containing 2.5% glutaraldehyde and 2% paraformaldehyde) at 4°C for 4 or more hours. Thereafter, the fixed material was washed with a phosphate buffer and was post-fixed with 2% osmium tetroxide (4°C, for 2 or more hours). After the fixed material had been subjected to a dehydration treatment, the resultant was embedded in an epoxy resin, and ultra-thin section specimens of 70 to 100 nm thickness were prepared. The thus obtained section specimens were double-stained with uranyl acetate and a lead citrate solution. The stained samples were observed under a transmission electron microscope.

A strain (e.g., NC67: Figure 1, right view), which was confirmed to completely lack capsules by electron microscopic observation, was selected as a S. uberis non-encapsulated strain. It is to be noted that four other S. uberis non-encapsulated strains were also obtained. Each of these five non-encapsulated strains was subjected to multi-locus sequence typing (MLST) (Coffey et al., Appl. Environ. Microbiol. 72: 1420-1428, 2006), and they were found to be different strains that were not genetically related to one another. Table 1 shows the MLST results of the above 5 non-encapsulated strains and the 3 encapsulated strains used in the Examples. According to the MLST of S. uberis, the criterion for being closely related is that 4 or more alleles are identical (see Pullinger et al., Appl Enviton Microbiol 72: 1429-1436, 2006; Watanabe et al., Jpn J Vet Res 669: 195-203, 2021; etc.). In light of these criteria, it became clear that the non-encapsulated strains obtained here are not closely related to one another.

**[Table 1] Table 1. Multilocus sequence typing (MLST) of non-encapsulated strains, and encapsulated strains exemplified in figu**

| Name of bacterial strain | Origin | MLST | | | | | | | | Presence or absence of capsule |
|---|---|---|---|---|---|---|---|---|---|---|
| | | Allele No. | | | | | | | ST | |
| | | *arcC* | *ddl* | *gki* | *recP* | *tdk* | *tpi* | *yqiL* | | |
| NIAH234RR | SCM | 3 | 1 | 3 | 4 | 9 | 1 | 3 | ST-1018 | Absence |
| 391B2 | DS | 26 | 18 | 4 | 10 | 105 | 6 | ND | Non-specific type | Absence |
| NC67 | CM | 4 | 2 | 56 | 3 | 3 | 2 | 3 | ST-1025 | Absence |
| NC103 | CM | 69 | 2 | 34 | 35 | 24 | 4 | 10 | ST-1043 | Absence |
| N11291 | CM | 3 | 2 | 3 | 2 | 5 | 2 | 3 | ST-1023 | Absence |
| NB38 | CM | 56 | 1 | 56 | 2 | 3 | 39 | 3 | ST-1038 | Presence |
| NH386 | CM | 1 | 1 | 2 | 2 | 17 | 2 | 3 | ST-1000 | Presence |
| NT9 | CM | 2 | 1 | 1 | 2 | 1 | 1 | 3 | ST-1016 | Presence |

| | | | | | | | | | | |
|---|---|---|---|---|---|---|---|---|---|---|
| ST: Sequence type CM: Clinical type mastitis milk; DS: Mammary gland secretion during dry lactation period; SCM: Latent mastitis milk NO: Not determined | | | | | | | | | | |

### 2. Preparation of vaccine antigen

The selected strain was allowed to proliferate by shaking culture, and was then subjected to sufficient centrifugal washing with PBS. The resultant strain was resuspended in PBS with 10% sucrose, and 1/3,300 (v/v) volume of β-propiolactone was then added thereto, followed by blending. The obtained mixture was incubated at 4°C for 24 hours for sterilization. After the S. uberis non-encapsulated strain had been sterilized, it was reacted at 37°C for 2 hours to inactivate β-propiolactone.

### 4. Antiserum against S. uberis non-encapsulated strain

### 4-1. Production of rabbit anti-S. uberis antiserum

### 4-1-1. Preparation of antigen

A S. uberis strain (an encapsulated strain or a non-encapsulated strain) was subjected to shaking culture in a Todd-Hewitt broth at 37°C for 18 to 20 hours, and was then centrifuged at 4°C and collected. Bacterial pellets were floated in a phosphate buffered saline (PBS) in an amount equal to the culture solution, and was centrifuged to remove the supernatant, so that the bacteria were washed (performed at 4°C, twice). The resultant was suspended in PBS with 10% sucrose in an amount of 1/30 of the culture solution, and β-propiolactone was then added thereto in an amount of 1/3,300 (v/v) of the bacterial suspension, followed by quickly blending. The thus obtained mixture was gently shaken at 4°C for 24 hours for sterilization. Subsequently, the reaction mixture was incubated at 37°C for 2 hours to inactivate β-propiolactone. Thereafter, the protein concentration of the dead bacterial antigen solution was measured (BCA protein assay), and was then appropriately diluted with PBS with 10% sucrose, as necessary. One-tenth of the amount of the produced dead bacterial antigen solution was applied onto a sheep blood agar medium, and was then cultured at 37°C for one day and one night to confirm that no viable bacteria remained in the medium.

### 4-1-2. Immunization and blood collection

The dead bacterial antigen solution (4 mg protein/mL) was mixed with an equal volume of aluminum hydroxide adjuvant (2% Alhydrogel adjuvant; Invivogen), and 250 µL of the antigen-adjuvant mixed solution per animal was subcutaneously injected into two sites on the back.

For partial blood collection, 5 mL of blood was aspirated from the ear vein with a syringe using a 22-gauge needle. Whole blood collection was performed after induction of deep anesthesia by aspirating blood from the heart with a syringe using a 18-gauge needle. Deep anesthesia was induced by intramuscular injection of 0.5 mL of a normal saline containing 0.5 mg of medetomidine hydrochloride, 2.0 mg of midazolam and 0.5 mg of butorphanol tartrate, per kg of body weight, into the waist according to the method of Konno et al. (Konno et al., Annual Report of Hatano Research Institute, 35: 53-59, 2012).

### 4-1-3. Experimental schedule

Immunization was performed three times at 2-week intervals. Partial blood collection was carried out before the initial immunization and 10 days after each immunization, and whole blood collection was carried out 14 days after the final immunization.

### 4-2. Production of bovine (heifer) anti-S. uberis non-encapsulated strain antiserum

### 4-2-1. Preparation of Antigen

Preparation of the antigen was carried out in the same manner as that for the preparation of the rabbit anti-S. uberis antiserum.

### 4-2-2. Immunization and blood collection

An antigen/adjuvant mixed solution consisting of 1.0 mL of dead bacterial antigen solution (8 mg protein/1.0 mL PBS with 10% sucrose) and 1.0 mL of DEAE-dextran solution (200 mg/mL PBS) was used per administration per cow. That is, 2.0 mL of the mixed solution was subcutaneously injected into the neck portion using a 20-gauge needle.

Partial blood collection was performed by aspirating 10 mL of blood from the jugular vein into a vacuum blood collection tube, using an 18-gauge needle. Serum was separated from the obtained blood. Whole blood collection was performed by releasing blood from the carotid artery, under deep anesthesia caused by intravenous injection with sodium pentobarbital.

### 4-2-3. Experimental schedule

Immunization was performed three times at 2-week intervals. Partial blood collection was carried out 7 days before the initial immunization, immediately before the initial immunization, and 7 days after the immunization. Whole blood collection was carried out 14 days after the final immunization.

### 4-3. Studies on antigen-binding ability of rabbit and bovine anti-S. uberis antisera

First, the binding ability of rabbit anti-S. uberis antibodies contained in the prepared antisera against various types of S. uberis strains (non-encapsulated strains and encapsulated strains) was studied. Rabbit anti-S. uberis antisera were added to plates, on which dead bacteria of various types of S. uberis strains (non-encapsulated strains and encapsulated strains) were immobilized, and the reactivity of the antibodies was confirmed by an ELISA method. The results are shown in Figure 2. Antisera prepared using 5 non-encapsulated strains (NC67, NIAH234RRR, 391B2, NC103, and NH291 strains) as antigens exhibited the same levels of reactivity to other non-encapsulated strains (i.e., non-encapsulated strains other than the non-encapsulated strain used as antigens) and to encapsulated strains (NB38, NH386, and NT9 strains). In contrast, antiserum prepared using an encapsulated strain as an antigen exhibited the highest reactivity to the bacterial strain used as an antigen, while the reactivity to other bacterial strains was low. These results show that antisera react to all of the S. uberis non-encapsulated strains that are not closely related to one another, at the same levels as the antisera reacting to other S. uberis strain including encapsulated strains (strains that are not antigens of the antisera) (see also Table 1).

In addition, when the same experiments as those described above were carried out with regard to bovine anti-S. uberis antisera that had been prepared using non-encapsulated strains such as NC67 and HIAH234RR as antigens, similar results were obtained (Figure 3). That is to say, the antigenic properties of the non-encapsulated strains and the encapsulated strains in bovines were each the same as those confirmed in the rabbits. That is, the antiserum against the non-encapsulated strains (NC67 and HIAH234RR) was highly reactive not only to the bacterial strain used as an antigen, but also to other bacterial strains, whereas the antiserum against the encapsulated strain was less reactive to bacterial strains other than the bacterial strain used as an antigen.

These results suggest that immunization of target animals with the S. uberis non-encapsulated strain can impart immunity to several different strains of S. uberis, without being affected by capsular antigenicity.

Subsequently, using bovine anti-S. uberis (non-encapsulated strain) antiserum, dead and viable bacteria of S. uberis encapsulated and non-encapsulated strains were immunostained, and thereafter, fluorescence microscopy was performed.

The dead bacteria were treated as follows. The dead bacterial solution prepared as described in "4-1-1. Preparation of antigen" was smeared on an anti-release agent (CREST)-coated glass slide (https://www.matsunami-glass.co.jp/product/glass/crest_glass_slide/), and was then immobilized in ethanol for 5 to 10 minutes. Thereafter, ethanol was removed, and the glass slide was rest at rest for about 1 minute for air drying. A blocking agent (Block Ace stock solution) was mounted, and was then left at rest for 45 minutes (Block Ace: https://www.saibou.jp/customer/pdf/catalog/blockace.pdf). After the glass slide had been gently rinsed with PBS, anti-S. uberis antiserum appropriately diluted with PBS containing 10% (v/v) Block Ace was mounted, and was then left at rest for 45 minutes. The diluted antiserum solution was removed from the glass slide, and the glass slide was then washed with PBS four times. Thereafter, DyLight 594-labeled rabbit anti-bovine IgG antibody (Bethyl Laboratories) appropriately diluted with PBS containing 10% (v/v) Block Ace was mounted, and was then left at rest for 40 minutes. The labeled antibody-diluted solution was removed, and the glass slide was then washed with PBS four times. DAPI solution (DOJINDO LABORATORIES) appropriately diluted with PBS was mounted, and was then left at rest for about 5 minutes. Thereafter, the DAPI solution was removed, and the glass slide was washed with PBS twice. The cells were enclosed with a cover glass, and were then observed under a fluorescence microscope.

On the other hand, viable bacteria were treated as follows. Bacteria were cultured by shaking as described in "4-1-1. Preparation of antigen," and were then collected. The bacteria were subjected to centrifugal washing with PBS (twice), and were then suspended in PBS to achieve an absorbance at 600 nm (optical path length: 1 cm) of 0.15, so that they were diluted. To 80 µL of the obtained bacterial suspension, 20 µL of bovine anti-S. uberis antiserum appropriately diluted with PBS was added, followed by blending, and the obtained mixture was then reacted at 37°C for 30 minutes. Thereafter, the bacteria were subjected to centrifugal washing with PBS (three times), and the resulting bacteria were then suspended in 80 µL of PBS. Thereafter, 10 µL of DyLight 594-labeled goat anti-bovine IgG antibody (Bethyl Laboratories) appropriately diluted with PBS was added to the suspension, followed by blending. After the obtained mixture had been reacted at 37°C for about 15 minutes, the bacteria were subjected to centrifugal washing with PBS (4 times), and were then suspended in PBS containing a small amount of 1% (w/v) egg white albumin, and the obtained suspension was smeared on an anti-release agent (CREST)-coated glass slide. The cells were enclosed with a cover glass, and were then observed under a fluorescence microscope.

The results of immunostaining against dead bacteria of the encapsulated strain (NH386) are shown in Figure 4. It was confirmed that an antibody prepared using the S. uberis non-encapsulated strain NC67 as an antigen binds to the surface of the encapsulated strain. Besides, an antibody against another non-encapsulated strain (NIAH234RR) also binds to the surface of the encapsulated strain. However, when antibodies against other encapsulated strains other than NH386 were used, the fluorescent staining image of the NH386 strain could not be obtained.

### 5. Confirmation of immunogenicity of S. uberis non-encapsulated strain in lactating cows and transition of antibody into milk

According to the same procedure as those described in 4-2-2 and 4-2-3 above, dead bacteria of S. uberis non-encapsulated strain were subcutaneously injected into the neck portion of milking cows with DEAE-dextran as an adjuvant three times at 2-week intervals. However, the amount of antigen/adjuvant injected was different from that used for immunization of heifers, and each cow was injected with a total of 3.0 mL of solution per injection, namely, with 1.5 mL of dead bacterial antigen solution and 1.5 mL of DEAE-dextran solution, as described in 4-2-2 above. Blood and milk samples were collected over time from the first week before initiation of immunization, and the anti-S. uberis IgG antibody titers in serum (2,000-fold dilution) and skimmed milk (100-fold dilution) were measured by ELISA. The results are shown in Figure 5. It was observed that the anti-S. uberis IgG antibody titer in serum was increased over time, and that the antibody titer in milk was also increased, as in the case of serum.

### 6. Infection protection test

### Test 1

With regard to the milking cow in 5 above, which had been immunized with the dead bacteria of the S. uberis non-encapsulated strain as an antigen, one week after the final immunization, 2 udders (infected udders; Figure 6) were inoculated with the S. uberis encapsulated strain (NH386) suspended in PBS (and the remaining 2 udders (control udders; Figure 6) were administered with control PBS), and thereafter, the number of bacteria and the number of somatic cells in the milk, the body temperature, a transition in the milk amount, and the like were observed. Infection of S. uberis into the udder was carried out by injecting the bacteria into the teat cistern, using a teat canal cannula. The number of bacteria inoculated into in the udder was 127 colony-forming units/5 mL PBS for control cow-1, 100 colony-forming units/5 mL PBS for control cow-2, and 512 colony-forming units/5 mL PBS for vaccinated cow-1 (per an udder in all of the cows). As controls, the same treatment as described above was carried out on non-immunized lactating cows. Transitions in the number of bacteria and the number of somatic cells in the milk are shown in Fig. 6. In the control cows (control cow-1 and control cow-2), both the number of somatic cells and the number of bacteria were increased over time in the milk of the udders inoculated with the S. uberis encapsulated strain. In contrast, in the cow immunized with dead bacteria of the S. uberis non-encapsulated strain (vaccinated cow), the number of somatic cells and the number of bacteria in the milk from the udders inoculated with the S. uberis encapsulated strain were hardly increased (i.e. infection does not hold and inflammation does not occur), or even if the number of bacteria and the number of somatic cells were temporarily increased (i.e. infection holds and inflammation occurs), the infection was confirmed to be quickly eliminated and the inflammation subsided.

In addition, the body temperature (rectal temperature) of the control cows inoculated with the S. uberis encapsulated strain fluctuated drastically, whereas that of the vaccinated cow showed almost no change (Figure 7). The milk amount of the control cows was decreased after the inoculation, whereas that of vaccinated cows was hardly changed at all (Figure 7).

### Test 2

Pregnant milking cows were subcutaneously immunized with dead bacteria of the S. uberis non-encapsulated strain, using DEAE-dextran as an adjuvant, into the neck portion thereof, at 35 and 14 days before the expected delivery date (actually, at 24 and 3 days before the actual delivery date due to early delivery). The amount of antigen/adjuvant inoculated was 3.0 mL in total, namely, 1.5 mL of the dead bacterial antigen solution and 1.5 mL of the DEAE-dextran solution described in 4-2-2 above, per cow. Forty-nine days after the final immunization, one udder (infected udder; Figure 8) was inoculated with 165 colony-forming units of the S. uberis encapsulated strain (NH386) suspended in 5 mL of PBS, and another udder (control udder; Figure 8) was inoculated with control PBS (5 mL) (the remaining two udders (not shown in the figure) were untreated). From 14 days before initiation of the immunization, blood was collected over time. In addition, colostrum was collected on the day of parturition, and thereafter, milk was collected over time. Anti-S. uberis IgA, IgG1 and IgG2 antibody titers in serum and skimmed milk were measured by ELISA, and then, while the antiserum prepared in 4-2-2 above was used as standard antiserum and the titers of each IgA, IgG1 and IgG2 specific antibodies in the antiserum were expressed as 100, the measured values were shown. As a result, as shown in Figure 8, it was observed that antigen-specific antibodies were induced in the blood and the milk, even in the case of immunization during pregnancy. Moreover, the number of somatic cells in the milk from the control udder and the infected udder temporarily exceeded 100,000 cells/mL after the inoculation, but from 1 or 2 days after the inoculation, the number of somatic cells remained less than 100,000 cells/mL, as before the inoculation (Figure 9). No bacteria were separated from all of the milk samples of the control udders, the infected udders, and the untreated udders throughout the test period.

From the aforementioned results, it became clear that administration of a S. uberis non-encapsulated strain as an immunogen can effectively protect against infection by different strains of S. uberis, without being affected by the presence or absence of a capsule.

### Industrial Applicability

Since the vaccine according to the present invention can protect against infection by Streptococcus species, in particular, by S. uberis, and can suppress the onset of mastitis, the present vaccine is expected to be utilized in the field of livestock industry.

## Claims

1. A vaccine comprising a non-encapsulated strain of Streptococcus species as an antigen.

2. The vaccine according to claim 1, which is **characterized in that** the antigen is a dead bacterium of the non-encapsulated strain or a portion thereof.

3. The vaccine according to claim 1 or claim 2, which further comprises a pharmaceutically acceptable adjuvant.

4. The vaccine according to any one of claims 1 to 3, which is for use in prevention of infection caused by Streptococcus species.

5. The vaccine according to claim 4, wherein the infection is mastitis.

6. The vaccine according to any one of claims 1 to 5, which is **characterized in that** the Streptococcus species is Streptococcus uberis (S. uberis).

7. An antibody that binds to both of a non-encapsulated strain and an encapsulated strain of Streptococcus species.

8. The antibody according to claim 7, which is **characterized in that** the antigen is a dead bacterium of the non-encapsulated strain of the Streptococcus species, or a portion thereof.

9. The antibody according to claim 7 or claim 8, which is **characterized in that** the Streptococcus species is S. uberis.

10. A method for preventing mastitis, comprising administering a pharmaceutically effective amount of a vaccine comprising a non-encapsulated strain of Streptococcus species as an antigen to a non-human mammal.

11. The method according to claim 10, which is **characterized in that** the Streptococcus species is S. uberis.
